# EUROPEAN PATENT APPLICATION

(11) **EP 3 501 513 A1**
(43) Date of publication of application: **26.06.2019**
(21) Application number: 17306888.3
(22) Date of filing: 21.12.2017
(51) Int. Cl.: A61K 31/17, A61P 9/00, A61P 43/00

(54) **USE OF CELIPROLOL FOR TREATING KYPHOSCOLIOTIC EHLERS-DANLOS SYNDROME**

(71) Applicant: Assistance Publique-Hôpitaux de Paris (AP-HP), 75004 Paris (FR)
(72) Inventor: FRANK, Michael, 78950 GAMBAIS (FR)
(74) Representative: Santarelli

(57) **Abstract**

The present invention relates to the use of celiprolol or a pharmaceutically acceptable salt thereof for treating kyphoscoliotic Ehlers-Danlos syndrome.

## Description

### FIELD OF THE INVENTION

The present invention pertains to the field of treatment of an orphan disease, namely the kyphoscoliotic Ehlers-Danlos syndrome (kyphoscoliotic EDS). More specifically, the invention pertains to the use of celiprolol or a pharmaceutically acceptable salt thereof for preventing cardiovascular events in kyphoscoliotic EDS patients in need thereof.

### BACKGROUND OF THE INVENTION

The kyphoscoliotic form of the Ehlers-Danlos syndrome (EDS; former EDS type VIA; OMIM#225400) is a rare autosomic recessive connective tissue disorder due to mutations of the lysyl-hydroxylase-1 gene (*PLOD1*)(Yeowell *et al*., 2000). The characteristic features of the disease are severe hypotonia at birth, progressive kyphoscoliosis, generalized joint hypermobility and scleral fragility that may lead to rupture of the ocular globe. The presence of three of these clinical criteria is suggestive of the disease and indicates laboratory testing (Beighton *et al*., 1998; Malfait *et al*., 2017). Further diagnostic criteria are tissue/skin fragility that may lead to atrophic scars and bruising, a marfanoid habitus, arterial rupture, microcornea, osteopenia and affected sibs (Yeowell *et al*., 1993). The prevalence of kyphoscoliotic EDS remains unknown. Estimates of incidence are 1:100 000 live-births with a carrier frequency estimated to be 1:150. Pathogenic variants of the *PLOD1* gene are characterized by a deficient activity of collagen lysyl-hydroxylase-1 (or procollagen-lysine 2-oxoglutarate 5-dioxygenase 1) leading to an underhydroxylation of collagen lysyl residues and ultimately to an impaired collagen crosslink formation. The diagnosis of kyphoscoliotic EDS can be made by measuring the ratio of urinary lysyl-pyridinoline to hydroxylysyl-pyridinoline (LP/HP) which is expected to be increased in case of a deficient lysyl-hydroxylase-1. Activity of the enzyme can also be tested in cultured skin fibroblasts (Krane *et al*., 1972). Formal diagnosis is also obtained by genetic testing of the *PLOD1* alleles. Kyphoscoliotic EDS has been associated with arterial fragility, but - unlike vascular EDS - arterial ruptures seem to be more prevalent than dissections (a specific type of arterial rupture in which the layers of the vessel separate prior to possible complete failure of the artery wall), and children seem particularly at risk.

Arterial fragility in kyphoscoliotic EDS remains poorly characterized and in absence of effective care, cardiovascular events cause an important morbidity and mortality in kyphoscoliotic EDS patients.

In 2010, Ong *et al.* reported the results of a clinical trial study for assessing the effects of celiprolol on prevention of cardiovascular events in another rare inherited disease called vascular Ehlers-Danlos syndrome (BBEST study). Vascular EDS is an autosomal dominant disorder resulting from heterozygous mutations in the *COL3A1* gene that cause structural defects in the pro-α(1) chain of type III procollagen. The resulting deficiency of structure of type III collagen affects the entire arterial tree, together with the skin and intestine.

Celiprolol is indicated for the management of mild to moderate hypertension and effort-induced angina pectoris. It has a unique pharmacology: it is a selective β1 receptor antagonist, but a β2 receptor partial agonist. It is also a weak α2 receptor antagonist. The BBEST study demonstrated decreased incidence of arterial rupture or dissection in vascular EDS patients taking celiprolol, compared to placebo.

Unlike in kyphoscoliotic EDS, arterial dissections (a specific type of arterial ruptures in which the layers of the vessel separate prior to possible complete failure of the artery wall) are prevalent in vascular EDS. Another difference regarding arterial fragility in these two diseases and is that children having a kyphoscoliotic EDS seem particularly at risk, which is not the case in vascular EDS. Different molecular mechanisms of both diseases may explain these differences in presentation and evolution: indeed, organ fragility in vascular EDS is the direct consequence of a structural weakness of the arterial wall, mainly due to a quantitative type III collagen deficiency (dominant negative effect), whereas kyphoscoliotic EDS is the result of a global enzymatic deficiency (loss of collagen hydroxylation).

The pathophysiology of vascular EDS is hence largely different from that of kyphoscoliotic EDS, so that the results obtained in the BBEST study could not be extrapolated to kyphoscoliotic EDS.

The present invention aims at fulfilling the unmet need for prevention of cardiovascular events in kyphoscoliotic EDS patients.

### SUMMARY OF THE INVENTION

The present invention pertains to the use of celiprolol or a pharmaceutically acceptable salt thereof for preventing cardiovascular events in kyphoscoliotic EDS patients in need thereof.

The invention also pertains to a method for determining if an individual having a kyphoscoliotic Ehlers-Danlos syndrome needs to be treated with celiprolol or a pharmaceutically acceptable salt thereof, comprising measuring the intima-media thickness of an elastic artery (carotid) in said individual, wherein a decreased intima-media thickness indicates that the patient needs to be treated with celiprolol to prevent cardiovascular events.

### DETAILED DESCRIPTION OF THE INVENTION

The present invention relates to a novel medical use of celiprolol or a pharmaceutically acceptable salt thereof, namely the treatment of kyphoscoliotic Ehlers-Danlos syndrome.

According to an embodiment, the present invention pertains to a method of treating kyphoscoliotic Ehlers-Danlos syndrome, comprising administering celiprolol or a pharmaceutically acceptable salt thereof to an individual in need thereof.

As used herein, the terms "treat", "treatment" and "treating" refer to any reduction of one or more symptom(s) associated with kyphoscoliotic EDS, such as, for example, a reduction of the occurrence and/or severity of cardiovascular accidents, and/or an increase in survival that results from the administration of celiprolol alone or combined with one or more other therapies.

Celiprolol (brand names Cardem®, Selectol®, Celipres®, Celipro®, Celol®, Cordiax®, Dilanorm®) is a medication in the class of beta blockers. Its chemical formula is N'-(3-Acetyl-4-(3-((1,1-dimethylethyl)amino)-2-hydroxypropoxy)phenyl)-N, N-diethylurea, its CAS number is 56980-93-9 and Drug Bank number is DB04846.

According to an embodiment of the present invention, celiprolol or a pharmaceutically acceptable salt thereof is administered to the individual at a dosage of at least 50 mg per day, preferably at a dosage ranging from 50 mg to 600 mg per day.

In adult kyphoscoliotic EDS patients, celiprolol or a pharmaceutically acceptable salt thereof is administered at a dosage superior (or equal) to 100 mg per day, preferably at a dosage ranging from 200 mg to 400 mg of celiprolol or a pharmaceutically acceptable salt thereof per day.

As mentioned above, children having a kyphoscoliotic EDS seem particularly at risk of cardiovascular accidents such as arterial ruptures. According to another aspect of the present invention, celiprolol or a pharmaceutically acceptable salt thereof is administered to a child having a kyphoscoliotic EDS. In particular, celiprolol or a pharmaceutically acceptable salt thereof can be administered to teenagers above 14 at a dosage similar to that used for adults. For younger patients, the skilled artisan will adapt the dosage and administration regimen depending on the patient's age, weight and tolerance. For example, dosages ranging from 50 mg once daily to 400 mg per day (200 mg bis in die) can be envisioned.

According to an embodiment of the present invention, celiprolol or a pharmaceutically acceptable salt thereof is administered twice daily.

According to an embodiment of the present invention, the treatment with celiproplol or a pharmaceutically acceptable salt thereof prevents cardiovascular events. In particular, the treatment with celiproplol or a pharmaceutically acceptable salt thereof prevents arterial rupture, arterial dissection and/or dissecting aneurysm.

According to an embodiment of the present invention, the individual has a history of arterial dissection or dissecting aneurysm.

According to an embodiment of the present invention, the individual has a decreased intima-media thickness of elastic arteries.

According to an embodiment of the present invention, the individual has a sibling with a history of arterial rupture, arterial dissection or dissecting aneurysm.

Another aspect of the present invention is a method for determining if an individual having a kyphoscoliotic Ehlers-Danlos syndrome needs to be treated with celiprolol or a pharmaceutically acceptable salt thereof, comprising measuring an intima-media thickness of an elastic artery in said individual. According to this method, a decreased intima-media thickness indicates that the patient needs to be treated with celiprolol or a pharmaceutically acceptable salt thereof to prevent cardiovascular events.

The above method can be performed for example by measuring the intima-media thickness of a carotid.

As exemplified in the experimental part below, the above method can be performed by measuring the intima-media thickness by high resolution echo tracking.

Other characteristics of the invention will also become apparent in the course of the description which follows of the biological assays which have been performed in the framework of the invention and which provide it with the required experimental support, without limiting its scope.

### EXAMPLES

### Case Report

A 41 year-old patient was referred to our department for suspicion of connective tissue disorder, following the occurrence of a spontaneous left tibial anterior artery dissection. The dissection was discovered in a context of acute calf pain without identified trauma. Complementary work-up of the arterial tree evidenced a dissecting aneurysm of the celiac trunk, with a flap extending to the superior mesenteric artery (SMA) (as a consequence of an anatomic variation, the SMA originated from the celiac trunk). Two-year prior arterial monitoring had not revealed any arterial defect. Her medical history was relevant for generalized muscle hypotonia at birth, bilateral congenital dislocation of the hip, delayed gross motor development (independent walking at the age of 9 years) and rapidly evolving kyphoscoliosis. Three ocular ruptures occurred at ages 21, 25 and 27 years, respectively. All ruptures occurred following minor trauma. The right eye ruptured twice, the left eye once only. Sympathic ophtalmia complicated the left ocular rupture and resulted in complete loss of vision of the left eye despite adequate treatment. Her family history was remarkable by the death at the age of 9-years of her only and younger sister, by spontaneous abdominal aortic rupture. Her phenotype was similar to her older sister's.

Physical examination evidenced major cutaneous fragility (bruising) and dystrophic scars. Skin elasticity was significantly increased associated with a soft, velvety consistency. She furthermore presented a severe kyphoscoliosis and diffuse joint hypermobility (Beighton score 8/9) with instability, particularly of the left shoulder (recurrent disclocations). Further osteoarticular signs were *pes planus* and minor *pectus excavatum.* Echocardiography revealed a minor dysplasia of the mitral valve (anterior leaflet), without significant prolapse. Notably, high resolution echotracking recorded an important reduction of the carotid intima-media thickness (453µm), and a consequent increase of steady circumferential wall stress (75kPa). Molecular analysis of *PLOD1* revealed a composite heterozygous deletion in exon 11 and 12 and deletion of exon 13 and 14 (c.[(1097+1_1098-1)_(1328+1_1329-1)del;(1470+1_1471-1)_(1650+1_1651-1)del]). Urinalysis showed a characteristic increase of the Deoxypyridinoline/Pyridinoline ratio to 10.4, confirming the diagnosis of kyphoscoliotic EDS. The anterior tibial artery aneurysm was managed medically and resulted in minimal sequellae at the site of dissection. The dissecting aneurysm of the celiac trunk also remained stable and a complete regression of the extension of the dissection to the SMA was noted. Two years later, the patient presented with similar pain in the left calf. Doppler ultrasound documented a left tibial anterior dissecting aneurysm. This second clinical event also resolved medically. Celiprolol was initiated and up-titrated to the optimally tolerated dose (400 mg/day) in prevention of further arterial events. The patient remained clinically silent to this day (3 years follow-up) and systematic arterial monitoring did not evidence further silent arterial defects.

### Discussion

Kyphoscoliotic EDS is a rare inherited connective tissue disorder, causing a vascular fragility. Indeed, patients with kyphoscoliotic EDS are at risk of spontaneous arterial rupture that may occur at any age, particularly during childhood. This early expression of arterial fragility seems more common than in patients with vascular EDS, which are typically free of symptoms during early childhood (Pepin *et al*., 2014; Frank *et al*., 2015). Our case corroborates these findings and illustrates well the potential severity of kyphoscoliotic EDS. Indeed, the proband's family tree suggests that her sister had the same condition and died at the age of 9 years by spontaneous abdominal aortic rupture. By contrast, the proband developed an acute arterial event at the age of 41 years only. The existence of such phenotypic variability within one family has been reported previously for other features, but not in relation with arterial accidents. The patient reported here is to our best knowledge the oldest reported kyphoscoliotic EDS patient, and the first with recurrent spontaneous dissections in medium size arteries without rupture. In regards of this extreme variability, vascular monitoring may be indicated at diagnosis, throughout the patient's life, both in probands and in affected sibs.

Due to the rarity of the disease and in the absence of large characterized patient cohorts, the incidence of arterial accidents in kyphoscoliotic EDS is unknown. However, the predominance of reports of traumatic arterial accidents highlights the exceptional vascular fragility of kyphoscoliotic EDS patients, and makes the occurrence of spontaneous, recurrent arterial accidents all the more likely. In 1989, Wenstrup *et al.* reviewed the clinical characteristics of n=10 patients with lysyl-hydroxylase deficiency. Ruptures of the femoral, intra-thoracic and vertebral arteries were reported (Wenstrup *et al*., 1989). Since the molecular characterization of kyphoscoliotic EDS in 2000, only two cases with molecularly proven pathogenic variants and arterial involvement have been reported (Rohrbach *et al*., 2011; Gok *et al*., 2012): the first with a coronary artery dissection during a coronarography in a 27 year-old man (reported as spontaneous), and a second with multiple arterial ruptures in medium size arteries starting at the age of 9 years. As for our patient, arterial dissection or dissecting aneurysm seems to be common in medium size arteries beside of the major risk of arterial rupture but they are not yet clearly a part of type VIA EDS definition in the current classification. We also note a fatal case of the superior vena cava rupture after extubation in an 8 year-old girl with molecularly proven disease, underlining again the particular vascular phenotype of the disease (*i.e.* including venous and arterial lesions) (Working *et al*., 2015). Published cases of vascular events in kyphoscoliotic EDS patients with sufficient characterization are reported in Table 1. Medicine-related illness and the risk of invasive procedures characterize the majority of reported cases (reporting bias). Spine surgery, likely because of its complexity and the length of the procedures, has a particularly high morbidity. However, in diagnosed patients, using a multidisciplinary approach in the management of the peri-operative period, minimal invasivity during surgery, the patient outcome may be significantly improved (Working *et al*., 2015).

Since patients with kyphoscoliotic EDS are exposed to acute, possibly life-threatening arterial accidents, it is of critical importance to determine whether it is possible to identify patients that are particularly at risk, and that may benefit from specific monitoring and medical intervention. The relationship between genotype and phenotype has never been evaluated for kyphoscoliotic EDS. The high resolution echotracking findings made in our patient suggest the existence of a detectable predisposition for arterial events. Indeed, a decreased intima-media thickness of elastic arteries was observed in our patient, suggesting that arteries of kyphoscoliotic EDS patients with arterial fragility are exposed to an increased arterial wall stress. Our patient benefited successfully from a treatment by celiprolol, without novel arterial manifestation over a four year follow-up.

### Conclusion

Patients with kyphoscoliotic EDS are not only exposed to life-threatening arterial ruptures, but also to spontaneous dissections of medium size arteries. Qualititative assessment of arterial fragility by high resolution echotracking of elastic artery properties may help identify patients/families that are particularly at risk. Patients with increased arterial risk, if not all patients with kyphoscoliotic EDS, may benefit from medical intervention to prevent arterial accidents.

**Table 1. Review of type VI Ehlers-Danlos syndromes with vascular events. ukn: unknown, A: arterial; V: venous; I: iatrogenic; S: spontaneous; T: traumatic**

| Patient | Sex | Age *(years)* | Diagnosis | | | | Type of accident | Circumstance of accident *(in order of occurrence)* | Lesion |
|---|---|---|---|---|---|---|---|---|---|
| | | | *compatible phenotype* | *urinary LP*/*HP elevation* | *cultured skin fibroblasts* | *molecular analysis* | | | |
| P1 (Heim *et al*., 1992) | M | 10 | + | ukn | + | ukn | V | I | Internal jugular vein ectasia post-catheterism |
| P2 (Rohrbach *et al*., 2011) | M | 27 | + | + | ukn | + | A | I | Spontaneous dissection of coronary arteries during coronary angiography |
| P3 (Esaka *et al*., 2009) | F | ukn | + | + | ukn | ukn | A | T | Right iliac artery rupture at delivery |
| P4 (Working *et al*., 2015) | F | 8 | + | ukn | ukn | + | V | I | Rupture of the superior vena cava after extubation |
| P5 (Debnath *et al*., 2007) | M | 20 | + | ukn | ukn | ukn | A | I | Coeliac trunk occlusion after spinal surgery (mechanism undisclosed) |
| P6 (Bush *et al*., 2014) | M | 32 | + | ukn | ukn | ukn | A/V | S/I/I/S/S | Profundal femoral artery rupture, femoral false aneurysm (access site), femoral venous rupture, splanchnic artery rupture (30 years), stroke (12 years) (mechanism undisclosed) |
| P7 (Yung *et al*., 2016) | M | 24 | + | ukn | ukn | ukn | A | T/S | Traumatic aortic dissection, popliteal artery aneurysm (age undisclosed) |
| P8 (Gok *et al*., 2012) | M | 12 | + | + | ukn | + | A | S/S/S | Brachial artery rupture, profundal femoral artery rupture (11 years) and iliac artery rupture (9 years) |
| P9 (Wenstrup *et al*., 1989) | ukn | ukn | + | ukn | + | ukn | A | ukn | Vertebral artery rupture |
| P10 (Wenstrup *et al*., 1989) | ukn | ukn | + | ukn | + | ukn | A | S/S | Multiple ruptures of the femoral artery and repeated spontaneous intrathoracic arterial ruptures |
| P11 (Akpinar *et al*., 2003) | F | 13 | + | ukn | + | ukn | A/V | I/I/I | Abdominal aorta and iliac artery rupture, common iliac vein rupture during spine surgery |
| P12 (Akpinar *et al*., 2003) | F | 13 | + | ukn | + | ukn | A | I | Intraoperative superior gluteal artery rupture (spine surgery) |

### REFERENCES

Akpinar S, Gogus A, Talu U, Hamzaoglu A, Dikici F. Surgical management of the spinal deformity in Ehlers-Danlos syndrome type VI. Eur Spine J Off Publ Eur Spine Soc Eur Spinal Deform Soc Eur Sect Cerv Spine Res Soc. 2003 Apr;12(2):135-40. (12)
Beighton P, Paepe AD, Steinmann B, Tsipouras P, Wenstrup RJ. Ehlers-Danlos syndromes: Revised nosology, Villefranche, 1997. Am J Med Genet. 1998 Apr 28;77(1):31-7.
Boutouyrie P, Germain DP, Fiessinger JN, Laloux B, Perdu J, Laurent S. Increased carotid wall stress in vascular Ehlers-Danlos syndrome. Circulation. 2004 Mar 30;109(12):1530-5.
Busch A, Suellner J, Anger F, Meir M, Kickuth R, Lorenz U, et al. Critical care of kyphoscoliotic type Ehlers-Danlos syndrome with recurrent vascular emergencies. Vasa. 2014 May;43(3):216-21.
Debnath UK, Sharma H, Roberts D, Kumar N, Ahuja S. Coeliac axis thrombosis after surgical correction of spinal deformity in type VI Ehlers-Danlos syndrome: a case report and review of the literature. Spine. 2007 Aug 15;32(18):E528-531.
Esaka EJ, Golde SH, Stever MR, Thomas RL. A Maternal and Perinatal Mortality in Pregnancy Complicated by the Kyphoscoliotic Form of Ehlers-Danlos Syndrome: Obstet Gynecol. 2009 Feb;113(2, Part 2):515-8.
Frank M et al. The type of variants at the COL3A1 gene associates with the phenotype and severity of vascular Ehlers-Danlos syndrome. Eur J Hum Genet. 2015 Dec;23(12):1657-64
Gok E, Goksel OS, Alpagut U, Dayιoglu E. Spontaneous Brachial Pseudo-aneurysm in a 12-year-old with Kyphoscoliosis-type Ehlers-Danlos Syndrome. Eur J Vasc Endovasc Surg. Nov 2012;44(5):482-4.
Heim P, Raghunath M, Meiss L, Heise U, Myllylä R, Kohlschütter A, et al. Ehlers-Danlos Syndrome Type VI (EDS VI): problems of diagnosis and management. Acta Paediatr. 1998 Jun;87(6):708-10.
Krane, SM, Pinnell, SR and RW Erbe. Lysyl-Protocollagen Hydroxylase Deficiency in Fibroblasts from Siblings with Hydroxylysine-Deficient Collagen, Proc. Nat. Acad. Sci. USA. 1972 Oct;69(10):2899-903.
Malfait F, Francomano C, Byers P, Belmont J, et al. The 2017 international classification of the Ehlers-Danlos syndromes. Am J Med Genet C Semin Med Genet. 2017 Mar;175(1):8-26.
Ong K-T, Perdu J, De Backer J, Bozec E, Collignon P, Emmerich J, et al. Effect of celiprolol on prevention of cardiovascular events in vascular Ehlers-Danlos syndrome: a prospective randomised, open, blinded-endpoints trial. Lancet. 2010 Oct 30;376(9751):1476-84.
Pepin MG, Schwarze U, Rice KM, Liu M, Leistritz D and Byers PH. Survival is affected by mutation type and molecular mechanism in vascular Ehlers-Danlos syndrome (EDS type IV). Genet Med. 2014 Dec;16(12):881-8.
Rohrbach M, Vandersteen A, Yi 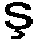 U, Serdaroglu G, Ataman E, Chopra M, *et al.* Phenotypic variability of the kyphoscoliotic type of Ehlers-Danlos syndrome (EDS VIA): clinical, molecular and biochemical delineation. Orphanet J Rare Dis. 2011;6:46.
Wenstrup RJ, Murad S, Pinnell SR. Ehlers-Danlos syndrome type VI: clinical manifestations of collagen lysyl hydroxylase deficiency. J Pediatr. 1989 Sept;115(3):405-9.
Working ZM, Hsiao M, Sanders JC, Bratton SL, D'Astous JL. Spontaneous Fatal Intraoperative Rupture of Great Vessel During Growing Rod Lengthening: Do Children With Ehlers-Danlos Syndrome Require the Availability of Vascular Expertise? A Case Report and Review of the Literature. J Pediatr Orthop. 2017 Jan;37(1):e4-e9
Yeowell HN, Steinmann B. Ehlers-Danlos Syndrome, Kyphoscoliotic Form. In: Pagon RA, Adam MP, Ardinger HH, Wallace SE, Amemiya A, Bean LJ, et al, editors. GeneReviews(®) [Internet]. Seattle (WA): University of Washington, Seattle; 1993-2017.
Yeowell HN, Walker LC. Mutations in the lysyl hydroxylase 1 gene that result in enzyme deficiency and the clinical phenotype of Ehlers-Danlos syndrome type VI. Mol Genet Metab. Oct 2000;71(1 2):212-24.
Yung MYH, Murray J, Thompson EC. Blunt aortic trauma in a patient with the Ehlers-Danlos syndrome type VI. J Surg Case Rep. 2016 Mar 7;2016(3).pii:rjw026.

## Claims

1. Celiprolol or a pharmaceutically acceptable salt thereof, for use in treating kyphoscoliotic Ehlers-Danlos syndrome.

2. Celiprolol or a pharmaceutically acceptable salt thereof, for the use of claim 1, wherein celiprolol or a pharmaceutically acceptable salt thereof is administered to an individual in need thereof at a dosage of at least 50 mg per day.

3. Celiprolol or a pharmaceutically acceptable salt thereof, for the use of claim 1 or claim 2, wherein celiprolol or a pharmaceutically acceptable salt thereof is administered to an individual in need thereof at a dosage ranging from 50 mg to 600 mg per day.

4. Celiprolol or a pharmaceutically acceptable salt thereof, for the use of any of claims 1 to 3, wherein celiprolol or a pharmaceutically acceptable salt thereof is administered to an individual more than 14 years old at a dosage ranging from 200 mg to 600 mg, preferably 200 mg to 400 mg of celiprolol or pharmaceutically acceptable salt thereof per day.

5. Celiprolol or a pharmaceutically acceptable salt thereof, for the use of any of claims 1 to 4, wherein celiprolol or a pharmaceutically acceptable salt thereof is administered twice daily.

6. Celiprolol or a pharmaceutically acceptable salt thereof, for the use of any of claims 1 to 3, wherein celiprolol or a pharmaceutically acceptable salt thereof is administered to an individual who is 14 years old or less, at a dosage ranging from 50 mg to 400 mg of celiprolol or pharmaceutically acceptable salt thereof per day.

7. Celiprolol or a pharmaceutically acceptable salt thereof, for the use of any of claims 1 to 6, wherein the treatment with celiproplol or a pharmaceutically acceptable salt thereof prevents cardiovascular events.

8. Celiprolol or a pharmaceutically acceptable salt thereof, for the use of any of claims 1 to 7, wherein the treatment with celiproplol or a pharmaceutically acceptable salt thereof prevents arterial rupture, arterial dissection and/or dissecting aneurysm.

9. Celiprolol or a pharmaceutically acceptable salt thereof, for the use of any of claims 1 to 8, wherein celiprolol or a pharmaceutically acceptable salt thereof is administered to an individual who has a history of arterial dissection or dissecting aneurysm.

10. Celiprolol or a pharmaceutically acceptable salt thereof, for the use of any of claims 1 to 9, wherein celiprolol or a pharmaceutically acceptable salt thereof is administered to an individual who has a decreased intima-media thickness of elastic arteries.

11. Celiprolol or a pharmaceutically acceptable salt thereof, for the use of any of claims 1 to 10, wherein celiprolol or a pharmaceutically acceptable salt thereof is administered to an individual who has a sibling with a history of arterial rupture, arterial dissection or dissecting aneurysm.

12. A method of treating kyphoscoliotic Ehlers-Danlos syndrome, comprising administering celiprolol or a pharmaceutically acceptable salt thereof to an individual in need thereof.

13. The method of claim 1, wherein celiprolol or a pharmaceutically acceptable salt thereof is administered to the individual at a dosage of at least 50 mg per day.

14. The method of claim 1, wherein celiprolol or a pharmaceutically acceptable salt thereof is administered to the individual at a dosage ranging from 50 mg to 600 mg per day.

15. The method of claim 1, wherein the individual is more than 14 years old and receives 200 mg to 600 mg, preferably 200 mg to 400 mg of celiprolol or a pharmaceutically acceptable salt thereof per day.

16. The method of claim 1, wherein celiprolol or a pharmaceutically acceptable salt thereof is administered twice daily.

17. The method of claim 1, wherein the individual is 14 years old or less and received 50 mg to 400 mg of celiprolol or a pharmaceutically acceptable salt thereof per day.

18. The method of claim 1, wherein the treatment with celiproplol or a pharmaceutically acceptable salt thereof prevents cardiovascular events.

19. The method of claim 1, wherein the treatment with celiproplol or a pharmaceutically acceptable salt thereof prevents arterial rupture, arterial dissection and/or dissecting aneurysm.

20. The method of claim 1, wherein the individual has a history of arterial dissection or dissecting aneurysm.

21. The method of claim 1, wherein the individual has a decreased intima-media thickness of elastic arteries.

22. The method of claim 1, wherein the individual has a sibling with a history of arterial rupture, arterial dissection or dissecting aneurysm.

23. A method for determining if an individual having a kyphoscoliotic Ehlers-Danlos syndrome needs to be treated with celiprolol or a pharmaceutically acceptable salt thereof, comprising measuring an intima-media thickness of an elastic artery in said individual.

24. The method of claim 12, comprising measuring the intima-media thickness of a carotid.

25. The method of claim 12, wherein the intima-media thickness is measured by high resolution echo tracking.
